# EUROPÄISCHE PATENTSCHRIFT

(11) **EP 1 687 238 B1**
(45) Veröffentlichungstag und Bekanntmachung des Hinweises auf die Patenterteilung: **09.12.2009**
(21) Anmeldenummer: 04798031.3
(22) Anmeldetag: 22.11.2004
(51) Int. Cl.: C01B 7/00

(54) **REAKTIVE CHLORVERBINDUNGEN, DEREN DERIVATE, ANIONEN UND SALZE SOWIE VERFAHREN ZU DEREN HERSTELLUNG UND VERWENDUNG**
REACTIVE CHLORINE COMPOUNDS, THE DERIVATIVES, ANIONS, AND SALTS THEREOF, METHOD FOR THE PRODUCTION THEREOF, AND USE THEREOF
COMPOSES CHLORES REACTIFS, LEURS DERIVES, ANIONS ET SELS, PROCEDES POUR LES PRODUIRE ET LEUR UTILISATION

(30) Priorität: 21.11.2003 DE 10354768
(43) Veröffentlichungstag der Anmeldung: 09.08.2006
(60) Teilanmeldung: 09012243.3
(73) Patentinhaber: CytoTools AG, 64285 Darmstadt (DE)
(72) Erfinder: WANNOWIUS, Klaus, Jürgen, 64372 Ober-Ramstadt (DE); KAISER, Dirk, 64859 Eppertshausen (DE)
(74) Vertreter: Schultheiss, Jürgen
(86) Internationale Anmeldenummer: PCT/EP2004/013212
(87) Internationale Veröffentlichungsnummer: WO 2005/049483

(56) Entgegenhaltungen:
- WO-A-00/48940
- DE-A1- 19 950 632

## Beschreibung

Die vorliegende Erfindung betrifft reaktive Chlorverbindungen wie Dichlorsäuren, das Zwischenprodukt Peroxochlorsäure sowie die peroxochlorige Säure und deren jeweilige Derivate, Anionen, und/oder Salze. Sie betrifft weiterhin Verfahren zur Herstellung dieser Verbindungen und deren Verwendung im pharmazeutischen und insbesondere im medizinischen Bereich als Arzneimittel und Desinfektionsmittel, sowie in der Kosmetik und der medizinischen Pflege als gewebeverträgliches Desodorans, sowie in den Bereichen Nahrungsmittelbehandlung und Technologie, insbesondere bei der Konservierung von Nahrungs- und Genussmitteln und als Bleichmittel sowie zur Trinkwasserdesinfektion, Entkeimung von Pflanzen und Früchten in der Landwirtschaft und als Oxidationsmittel in der technischen Chemie sowie zur Abgasreinigung.

Oxidationsmittel finden vielfältigste Anwendungen in der technischen Chemie, in der Hygiene und Nahrungsmittelkonservierung, in der Kosmetik und auch in der Pharmazie.

Nach Polly Matzinger (Polly Matzinger: "Tolerance, Danger, and the Extended Family" in Annu. Rev. Immunol. 1994, 12) senden gewaltsam, d.h. durch massive Strahleneinwirkung, toxische Substanzen, parasitäre, bakterielle oder virale Infektionserreger, lytisch, nichtapoptotisch, sterbende Zellen Gefahrensignale ("danger signals") aus, welche andauern müssen, damit die körpereigene Abwehrkraft, die neben dem eigentlichen Antigensignal einer unspezifischen Costimulation durch Antigen-präsentierende Zellen (z.B. Makrophagen) bedarf, klinisch optimal wirksam werden kann.

Bei einem gewaltsamen, nicht-apoptotischen Zelluntergang sind Fresszellen (sogenannte Mikro- und Makrophagen) für die Zelltrümmerbeseitigung verantwortlich. Bei dieser Zelltxümmerbeseitigung werden oxidativ wirksame Sauerstoffmetabolite freigesetzt. Wasserstoffperoxid (H₂O₂) ist der bekannteste Vertreter. *In-vitro*-Versuche zeigen, dass H₂O₂ im mikromolaren Bereich über Aktivierung des Transkriptionsfaktors NF-kappa B zu einer Immunmodulation von Lymphozyten führen kann (R. Schreck et al., The EMBO Journal 10(8), 2247-58 (1991); M. Los et al., Eur. J. Immunol. 25, 159-65 (1995). Der Arbeitskreis von Avraham Novogrodsky war der erste, der *in vitro* gezeigt hat, dass bestimmte Oxidantien (Bowers W.E.: "Stimulation of Lymphocytes with periodate or Neuraminidase plus Galactose Oxidase - NAGO" p. 105 - 109, Review in Immunochemical Techniques Part K Methods in Enzymology Vol. 150, 1987) unter anderem auch das im Körper selber gebildete H₂O₂ comitogen die durch Antigenreiz hervorgerufene Lymphozytenvermehrung steigern, wenn sich gleichzeitig Makrophagen in der Lymphozytenkultur befinden (Stenzel K.H., Rubin A.L., Novogrodsky A.: "Mitogenic and Comitogenic Properties of Hemin." J. Immunol. 127, 6: 2469-2473 et ibid. cit. ref.). Werden die oxidativ wirksamen Sauerstoffmetabolite im Körper in nicht ausreichendem Maße gebildet, bleibt eine Immunabwehr unvollkommen bzw. sie bleibt sogar ganz aus, so dass eine Toleranz oder pathologische Anergie entsteht. Werden sie überschießend bzw. schwelend, übermäßig langanhaltend produziert, dann entstehen chronische Entzündungen und Gewebsvemarbungen.

Es ist nach diesen Befunden davon auszugehen, dass oxidativ wirksame Sauerstoffverbindungen besonders in solchen klinischen Situationen eine therapeutische Wirkung haben, in denen ihre körpereigene Bildung mangelhaft bleibt bzw. nachlässt, bevor die Körperschäden vollkommen wiederhergestellt und die Infektionserreger vollständig beseitigt wurden. Es ist besonders in den Fällen mit einem Behandlungserfolg zu rechnen, in denen die Erreger die Zellen zwar infizieren, sie aber nicht zerstören, so dass die "danger signals" ausbleiben. Beispielhaft seien hier Infektionen mit Lepra- und Tuberkelbazillen und solche mit Herpes- und AIDS (HIV) -Viren genannt.

Über den erfolgreichen klinischen Einsatz von Kaliumbichromat zur Abheilung von jauchenden chronischen Wunden wurde bereits 1906 berichtet (Fenwick, J.: "The Treatment of Cancer by the Use of Potassium Bichromate", British Medical Journal, March 6th, 1909, 589 - 591).

Zahlreiche weitere, in der Zwischenzeit erschienene Veröffentlichungen zeigen, dass in physiologischer Weise im Körper gebildetes Wasserstoffperoxid wie auch das *in vivo* noch kurzlebigere Peroxonitrit, das aus dem ebenfalls physiologischen Nitroxid und Wasserstoffperoxid entstehen kann, ebenfalls Wundheileffekte zeigen, an welchen eine positive Immunmodulation essentiell beteiligt ist. Beispielsweise beschreibt die EPA-0390829 eine Methode zur Steigerung der syngenen intradermalen Zellvermehrung durch Wachstumsfaktoren beim Menschen mit Wasserstoffperoxidinjektionen. Eine solche comitogene Steigerung der Wachstumsfaktorwirkung von Interleukin-2 wurde 1987 auch für Periodat beschrieben (Wang J. et al., The American Journal of Medicine 1987, 83: 1016 - 1023).

Es hat sich gezeigt, dass (co)mitogene Oxidantien nicht tolerable Nebenwirkungen haben, wie z.B.: bei Bichromat: die inzwischen bekannt gewordene krebserzeugende Wirkung von Chromoxid. Bei Periodat: Iodüberempfindlichkeit und toxische Wirkung. Deswegen muß der klinische Einsatz umständlich als "adoptive transfer" stattfinden, d.h. die Blutzellen werden entnommen, *in vitro* behandelt und dann *in vivo* zurückgegeben, wie in der zuvor zitierten Arbeit von J. Wang et al. 1987 beschrieben. Bei NAGO: die Fremdproteinsensibilisierung. Bei H₂O₂: die Bildung toxischer Sauerstoffradikale. Auch stehen ihrer Verwendung als Arzneimittel technische Probleme entgegen, z.B.: bei H₂O₂: geringe Haltbarkeit in verdünnter wässriger Lösung; die Katalaseempfindlichkeit mit massiver Sauerstoffgasfreisetzung. Bei oxidierten Ubichinon-Derivaten: Galenische Probleme und eingeschränkte Bioverfügbarkeit.

Es war daher bisher nicht möglich, die experimentell nachgewiesene immunpharmakologische Wirkung (co)mitogener Oxidantien auf die Geweberegeneration/Wundheilung, auf die Infektabwehr bzw. auf die Steigerung der Immunantwort auf die klinische Praxis zu übertragen, in der neben einer örtlichen Anwendung auch eine systemische Anwendung meist in Form einer intravenösen Verabreichung wünschenswert ist.

Durch elektrochemische Oxidation behandelt Theo Gilbert Hinze (US 20030133878, "Composition for the treatment of legionella pneumophila and a method for such treatment") wässrige Lösungen von NaCl oder KCl₂ (vermutlich handelt es sich im letzteren Fall um einen Druckfehler) bei pH 6,5 - 7,5. Man vermutet, dass neben anderen Ionen das bis dahin nur in der vorangegangenen Erfindung beschriebene Cl₂O₆²⁻-Ion vorhanden sein könnte. In diesem Dimeren liegen die Chloratome in den Wertigkeitsstufen +3 und +5 vor.

In der Patentliteratur sind einige weitere Chlor-Sauerstoff-Präparationen beschrieben, die insbesondere in solchen technischen Bereichen Verwendung finden, in denen sie als Oxidantien nicht nur in der Industrietechnik als Bleichmittel und Geruchsbeseitiger eingesetzt werden, sondern auch zur paramedizinischen Verwendung empfohlen werden, wie in der Kosmetik zur Haut und Haarpflege, zur Haushaltspflege, im sanitären Sektor zur Hygiene und/oder als Desinfektionsmittel von Oberflächen (US 2,701,781; US 3,123,521) und/oder Wunden (US 4,084,747; EP-A-0 744 895), als Konservierungsmittel für Käse (US3,147,124), zur Trink- und Badewasser-Aufbereitung (US 4,296,103; DE-A-44 05 800, DE-A-19 518 464; WO 96/33947; WO 97/06098). Die US 4,296,103, EP-A-0 136 309, US 4,507,285 und EP-A-0255145 beschreiben die Arzneimittelanwendung von Chlor-Sauerstoff-Präparationen.

In der WO 00/48940 wurde über die Bereitstellung eines Chlorhydroperoxids mit der Formel HOOClO₂ berichtet, in der Chlor 5-wertig ist. Dieses Hydroperoxid verhält sich als Säure, die in wässrigem Milieu das Anion O₂ClOO⁻ liefert. Es wurde daher als Peroxochlorsäure und sein Anion als Peroxochlorat bezeichnet. Es wird berichtet, dass die Vereinigung zweier Peroxochlorationen unter Abspaltung eines Sauerstoffinoleküls zu Abkömmlingen (Derivaten) des Peroxochlorats mit einer Peroxogruppierung und zwei Chloratomen mit unterschiedlicher Wertigkeit führen kann. Diesem Ionen wird die Summenformel (Cl₂O₆)²⁻ zugeschrieben.

Es wird offenbart, dass es möglich sei, stabile Peroxochlorsäure und stabile Salze bzw. Anionen davon in Lösung herzustellen. Beispielsweise werden diese Verbindungen durch Umsetzung von Chlordioxid mit Wasserstoffperoxid in wässriger Lösung erhalten, wenn bei pH-Werten von gleich oder oberhalb des pKs-Wertes von Peroxochlorsäure (HOOClO₂) gearbeitet wird. Bevorzugt wird bei pH-Werten von 6,5 und darüber, besonders bevorzugt im pH-Wert Bereich 10 - 12 gearbeitet.

In der WO 00/48940 werden somit Peroxochlorsäure oder deren Salze, Peroxochlorsäure und deren Salze bzw. Anionen in wässriger Lösung, oligomere Derivate des Peroxochlorats mit gemischt-valenten Chloratomen und deren Salze bzw. Anionen in wässriger Lösung sowie das Kohlendioxidaddukt als Säure, als Anion in Lösung, oder als Salz offenbart.

Es hat es sich zwischenzeitlich gezeigt, dass eine Isolierung eines kristallinen Metall-Salzes des Peroxochlorats nach der in der WO 00/48940 angegebenen Vorschrift nicht gelingt.

Aufgrund der geringen Konzentrationen an Peroxochlorat in den nach den dort offenbarten Vorschriften hergestellten Präparationen gelingt auch die Darstellung der Desoxodimere in nur untergeordnetem Maße.

Svensson und Nelander offenbaren in J. Phys. Chem. A 1999, 103, 4432-4437 die Darstellung von HOOClO₂ bei tiefen Temperaturen von 17K (-256,15° C).

Sämtliche beschriebenen Chlor-Sauerstoff-Präparationen erfüllen daher die Anforderungskriterien einer modernen Arzneimittelzulassung nicht, die besagen, dass die Pharmakodynamik der Präparation einer chemisch definierten Verbindung als sogenannter Wirksubstanz zugeordnet werden können muß, auf die das Arzneimittelprodukt zu standardisieren ist, nicht zuletzt, um so eine gleichbleibende Arzneimittelqualität zu gewährleisten.

Die an sich guten Chlor-Sauerstoffverbindungen der WO 00/48940, und insbesondere das dort offenbarte Desoxodimer lassen sich jedoch bisher nur in untergeordnetem Maße herstellen, so dass eine wirtschaftliche Verwertung unmöglich erscheint.

Aufgabe der Erfindung war es daher, Oxidationsmittel bereitzustellen, die die vorstehenden Nachteile nicht aufweisen. Die Oxidationsmittel sollen neben der üblichen Anwendbarkeit für Oxidationszwecke auf technischem und medizinischem Gebiet, sowie zur Desinfektion, auch die Möglichkeit zur Formulierung als Arzneimittel zur örtlichen, wie auch systemischen Anwendung, z.B. zur intravenösen Verabreichung bieten, beispielsweise als Arzneimittel für die Geweberegeneration, die Wundheilung und zur Infektabwehr bzw. zur Steigerung der Immunantwort. Es soll darüber hinaus den Anforderungen der modernen Arzneimittelzulassung genügen.

Insbesondere war es daher Aufgabe vorliegender Erfindung, weiter verbesserte Oxidationsmittel sowie verbesserte Verfahren zu deren Herstellung und deren Verwendung bereitzustellen.

Gelöst wird diese sowie weitere nicht explizit genannte Aufgaben, die sich jedoch aus dem einleitend genannten Stand der Technik ohne weiteres ergeben, durch die in den Ansprüchen definierten Ausführungsbeispiele der Erfindung.

Übenaschenderweise hat sich gezeigt, dass diese Aufgabe durch die Bereitstellung von reaktiven Chlorverbindungen wie Dichlorsäuren, dem Zwischenprodukt Peroxochlorsäure sowie der peroxochlorigen Säure sowie deren jeweiligen Derivaten, Salzen und Anionen gelöst werden kann.

Die erfindungsgemäßen neuen Dichlorsäuren sind in der folgenden Tabelle 1 gezeigt. Von diesen Dichlorsäuren stellen die Dichlorsäuren Nr. 1 bis Nr. 3 besonders bevorzugte Ausführungsformen der vorliegenden Erfindung dar.

**Tabelle 1 :**

| **Nr.** | **Formale Oxidationsstufen des Chlor** | **Strukturformel der Säure** | **Strukturformel des Di-Anions** |
|---|---|---|---|
| **1** | +5, +5 | 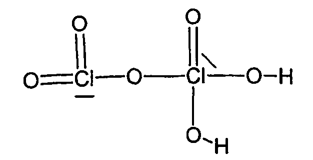 | 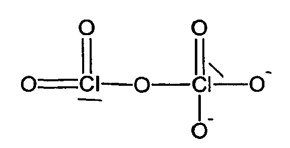 |
| **2** | +6, +4 | 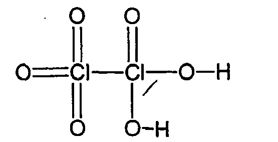 | 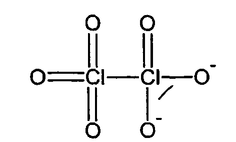 |
| **3** | +5, +5 | 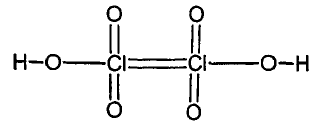 | 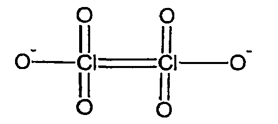 |
| **4** | +5, +3 | 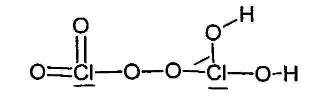 | 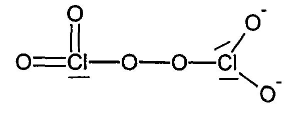 |

Neben den bereits beschriebenen Wertigkeitspaaren +3/+5 (WO 00/48940) und +4/+4 (Bogdanchikov G.A., Kozlov, Y.N. and Berdnikov, V.M. "The Mechanism of the Elementary Act of HO2-Anion Oxidation by a ClO2 Radical in Aqueous Solution" Khim.Fiz. 1983 (5), 628-636)) können die erfindungsgemäßen Dichlorsäuren Nr. 1 bis Nr. 3 mit den Wertigkeiten von +6/+4 und +5/+5 für Chlor mit dem erfindungsgemäßen Verfahren zum erstenmal hergestellt werden. Das Anion der Säure der Nr. 4 wird in der WO 00/48940 beschrieben. Das dort beschriebene Herstellungsverfahren funktioniert jedoch nicht.

In der WO 00/48940 wurde postuliert, dass sich aus 2 Molekülen einer reaktiven Chlor-Sauerstoff Spezies (Peroxochlorat) über die Reaktion

2 ⁻OOClO₂ → Cl₂O₆²⁻ + O₂

das Desoxo-Dimere bildet, bei dem die Chloratome in den Oxidationsstufen +3 und +5 vorliegen. Eine arzneimittelrechtlich wünschenswerte Herstellung einer stabilen Verbindung nach Beispiel 6 der WO 00/48940 gelingt jedoch nicht.

Die Entstehung des dimeren Derivats aus 2 Molekülen Peroxochlorat nach der Gleichung

2 ⁻OOClO₂ → Cl₂O₆²⁻ + O₂

ist nämlich nur bei sehr hohen Konzentrationen an Peroxochlorat (etwa ab 2 bis 3 mol/L) zu erwarten. Solche hohen Konzentrationen sind jedoch wegen der hohen Instabilität der Verbindung praktisch nicht darstellbar.

Die Untersuchungen, die zur vorliegenden Erfindung geführt haben, zeigen jedoch, dass die Umsetzung von Peroxochlorat-Ionen O₂ClOO⁻ mit Chlorit-Ionen (ClO₂-) überraschenderweise direkt zu der Palette der "dimeren" Cl₂O₆²⁻-Spezies führt:

O₂ClOO⁻+ ClO₂⁻→ Cl₂O₆²⁻ -> -> und Isomere

Weiterhin zeigte sich überraschenderweise, dass mit Hilfe des erfindungsgemäßen Verfahrens auch die Bereitstellung des bislang unbekannten Peroxochloritions, O=ClOO⁻ und der davon abgeleiteteten peroxochlorigen Säure O=ClOOH insbesondere in den erfindungsgemäßen, Chlorit-enthaltenden Lösungen gelingt.

Diese Chlorverbindungen wurden bislang noch nicht beschrieben.

Besonders um den Neutralpunkt herum tritt der Zerfall der Dichlorspezies Cl₂O₆²⁻ in Chlorationen ClO₃⁻ und Peroxochlorit-Ionen OClOO⁻ in deutliche Konkurrenz zu den beschriebenen intramolekularen Redoxreaktionen der Dichlorspezies, die zu den Verbindungen 1-4 in obiger Tabelle führen.

Sofern in der vorliegenden Offenbarung von Anionen die Rede ist, ist die Gegenwart erforderlicher Gegenionen (vor allem in Lösung) eingeschlossen. Es soll mit der Bezeichnung Anionen vor allem zum Ausdruck gebracht werden, dass in Lösung das Dichlorat die stabilere Form gegenüber der protonierten Säure ist. Allerdings kann erfindungsgemäß je nach Zusammenhang der Begriff "Anion" auch stellvertretend für die Säure stehen, der Begriff "Säure" kann ebenso stellvertretend für das "Anion" stehen.

Die Erfindung betrifft auch das Verfahren zur Herstellung von Zubereitungen, die die Dichlorsäuren bzw. deren Derivate, Anionen und/oder Salze und/oder die erfindungsgemäße peroxochlorige Säure bzw. deren Derivate, Anionen und/oder Salze enthalten.

Dadurch, dass man
(a) Chlordioxid mit einer wässrigen oder wasserhaltigen Lösung von Wasserstoffperoxid oder einem anderen Hydroperoxid oder Peroxid bei einem pH-Wert von ≥ 6,5 umsetzt,
(b) den pH-Wert durch Zusatz einer Säure auf 3 bis 6, erniedrigt,
(c) die gasförmige freie reaktive Chlorverbindung, bevorzugt die protonierte Peroxochlorverbindung mit einem gekühlten Gas austreibt und in einer basischen Lösung mit einem pH-Wert von >10 auffängt,
(d) die aufgefangene freie reaktive Chlorverbindung, bevorzugt die Peroxochlorverbindung bei einem pH zwischen 6 und 8, bevorzugt etwa 7 mit einem bis zu 100fachen Überschuss, bevorzugt bis zu 10fachen Überschuss Chlorit inkubiert,
gelingt es auf erstaunlich einfache Art und Weise, die erfindungsgemäßen Dichlorsäuren herzustellen.

Die erfindungsgemäßen Dichlorsäuren und auch die bei physiologischen pH-Werten vorhandenen Anionen können daher erfindungsgemäß auch als Gemisch mit Peroxochlorat und Chlorit in Lösung vorliegen. Eine solche, die erfindungsgemäßen Dichlorsäuren, peroxochlorige Säure, Peroxochlorat sowie Chlorit umfassende Lösung zählt daher zu den besonders bevorzugten Ausführungsbeispielen der vorliegenden Erfindung, und ist in Anspruch 2 unter Schutz gestellt.

In der WO 00/48940 wurden dagegen Chlorit-freie Lösungen erzeugt, in denen die Dichlorsäuren und die peroxochlorige Säure der vorliegenden Erfindung nicht vorhanden sind, oder es wurden chlorithaltige Präparationen erzeugt, die praktisch nur Chlorit enthielten, so dass sie für einen pharmazeutischen Einsatz ungeeignet sind.

Da große Mengen an Chlorit jedoch einer Verwendung der erfindungsgemäßen Dichlorsäuren im pharmazeutischen Sektor abträglich sind, ist es insbesondere bevorzugt, wenn in dem Endprodukt der erfindungsgemäßen Lösungen Chlorit in nicht mehr als 20fachem, bevorzugt nicht mehr als 5fachem, und insbesondere nicht mehr als im 3fachen Überschuss in Gewichtsanteilen bezogen auf das Gesamtgewicht der Lösung vorliegt.

Insbesondere liegen die erfindungsgemäßen Dichlorsäuren und die peroxochlorige Säure in dieser Lösung in Mengen von etwa 0,1-20 Gew.-%, bevorzugt 3-5 Gew.-% vor, bezogen auf den Gewichtsanteil eingesetztes ClO₂. Der qualitative Nachweis gelingt über RamanSpektroskopie. Die Durchführung dieser Art von Spektroskopie ist dem Fachmann auf dem Gebiet selbstverständlich. Die erhaltenen Spektrogramme unterscheiden sich deutlich von denen, die mit dem Verfahren der WO 00/48940 erhalten werden. Die Bestimmung des quantitativen Anteils kann über Titration erfolgen.

Ein weiterer qualitativer Nachweis ist über die Reaktion mit dem Häm-Eisen möglich. In Gegenwart der erfindungsgemäßen Dichlorsäuren verläuft der zeitliche Verlauf der Änderung der Intensität der Soret-Bande deutlich anders als mit den Lösungen, die mit dem Verfahren aus der WO 00/48940 erhalten wurden.

Das erfindungsgemäße Verfahren besteht darin, Chlordioxid mit wässrigen oder wasserhaltigem Wasserstoffperoxid oder einem anderen dem Fachmann geläufigen Peroxid oder Hydroperoxid, wie z.B. Peroxocarbonat oder Perborat oder dem Harnstoffaddukt des Wasserstoffperoxids bei einem pH-Wert von 6,5 oder höher, bevorzugt pH 10-12 umzusetzen. Es ist bevorzugt, den pH-Wert auf einem konstanten Wert zu halten.

Darüber hinaus hat es sich überraschenderweise gezeigt, dass die als Zwischenprodukt auftretende Peroxochlorsäure und ihre Anionen und Derivate auch durch die Umsetzung von Chlordioxid mit anderen Oxidationsmitteln, die die Peroxogruppierung enthalten, erhalten werden können.

Die Umsetzung kann in wässrigem Milieu oder in wasserhaltigem Milieu durchgeführt werden. Es können beispielsweise neben Wasser auch mit Wasser mischbare Lösungsmittel vorliegen, wie beispielsweise Alkohole, wie z.B. Alkanole, wie Methanol, Ethanol oder dergleichen, oder Mischungen davon.

Wahlweise kann auch von anderen Chloroxiden ausgegangen werden. So lässt sich beispielsweise Chlormonoxid vorzugsweise in seiner dimeren Form (Cl₂O₂) ebenfalls mit einem Hydroperoxid (vorzugsweise Wasserstoffperoxid) zum gewünschten Produkt umsetzen. Die Umsetzung gelingt im gleichen pH-Gebiet wie beim Chlordioxid angegeben.

Die Reaktionstemperatur kann erhöht werden, beispielsweise bis zu etwa 50°C; bei rein wässrigen Systemen liegt die niedrigste Temperatur bevorzugt bei etwa 0°C. Mit Chlordioxid sollte man allerdings nicht unter +10 Grad Celsius arbeiten, da sich unterhalb dieser Temperatur das Chlordioxid-Gas verflüssigt und es zu Verpuffungen kommen kann. Liegen zusätzliche organische Lösungsmittel und/oder hohe Konzentrationen der beteiligten Reagenzien vor, so können auch niedrigere, d.h. unter dem Gefrierpunkt von Wasser liegende Temperaturen verwendet werden. Bevorzugt wird bei Raumtemperatur gearbeitet.

Das zur Umsetzung benötigte Chlordioxid steht dem Fachmann zur Verfügung und kann in üblicher Weise hergestellt werden. Beispielsweise kann es hergestellt werden durch Reaktion eines Chlorits mit einer Säure (beispielsweise Natriumchlorit mit Schwefelsäure) oder durch Reduktion von Chlorat, beispielsweise mit schwefeliger Säure.

Das so erhaltene Chlordioxid kann gegebenenfalls nach Entfernung von vorhandenen Spuren von Chlor in an sich bekannter Weise (Granstrom, Marvin L.; and Lee, G. Fred, J. Amer. Water Works Assoc. 50, 1453-1466 (1958)) befreit werden.

Sollte das zur ClO₂-Herstellung eingesetzte Chlorit mit Carbonat verunreinigt sein, so entsteht ClO₂ das mit CO₂ verunreinigt ist, und/oder die in der WO 00/48940 beschriebenen Kohlensäureaddukte. Zur Absorption des Kohlendioxids sollte der Chlordioxid- und Kohlendioxid-haltige Gasstrom durch eine mit Lauge beschickte Waschflasche geleitet werden. Bei kurzen Kontaktzeiten wird zwar das CO₂ aber nicht das ClO₂ von der Lauge absorbiert. Besser ist es jedoch, die Verunreinigungen an Carbonat durch fraktionierte Kristallisation des eingesetzten Natriumchlorit zu befreien. Eine Verunreinigung des Peroxochlorats mit Carbonat lässt sich leicht im Ramanspektrum erkennen. Anstelle der scharfen Bande bei 1051 cm⁻¹ erhält man eine Doppelbande bei 1069 cm⁻¹ (breit) und die im Rahmen der Erfindung wichtige Bande bei 1051 cm⁻¹ (scharf).

Das Chlordioxid kann mit einem Inertgas, wie Stickstoff oder einem Edelgas, wie Argon, jedoch auch durch Luft oder Sauerstoff zur Reaktion mit der Peroxo-Verbindung oder des Hydroperoxids wie z.B. dem Wasserstoffperoxid oder Percarbonat oder Perborat gefördert werden. Beispielsweise ist es möglich, das Chlordioxid in einem ersten Reaktionsgefäß herzustellen und mit den genannten Gasen oder einem Gemisch daraus in ein zweites Reaktionsgefäß einzuleiten, in dem sich die Peroxo-Verbindung (Peroxid oder Hydroperoxid) in wasserhaltiger oder wässriger Lösung befindet.

Der pH-Wert des Reaktionsgemischs wird durch Zugabe einer Base gleich oder oberhalb 6,5 gehalten. Bevorzugt ist es, den pH-Wert konstant zu halten. Dies kann entweder manuell oder auch automatisch durch ein "pH-Stat"-Gerät erfolgen.

Als Basen können übliche anorganische oder organische Basen, wie Alkalilaugen, beispielsweise Natronlauge oder Kalilauge oder Erdalkalihydroxide, Ammoniak oder organische Basen, wie Stickstoffbasen, dienen. Es können auch die Hydroxide von quaternären Ammoniumsalzen, insbesondere Alkyl-, wie Trialkyl- oder Tetraalkylammoniumhydroxide, oder Zinkhydroxide eingesetzt werden.

Der Gehalt an Hydroperoxid in dem Reaktionsgemisch kann beispielsweise durch potentiometrische Titration mit einer Säure, wie beispielsweise Salzsäure, bestimmt werden.

Die nach dem vorstehend beschriebenen Verfahren erhaltenen Lösungen können als solche oder auch in abgewandelter Form zum Einsatz gebracht werden. Beispielsweise kann überschüssiges Wasserstoffperoxid in üblicher Weise, z.B. mit einer Schwermetallverbindung, wie Braunstein, beseitigt werden. Analog können Überschüsse der anderen Oxidationsmittel beseitigt werden.

Überschüsse an Chlordioxid (ClO₂) können mit H₂O₂ beseitigt werden. Dies sollte möglichst bald geschehen, da ansonsten über

**2 ClO₂ + 2OH⁻ -> ClO₂⁻ + ClO₃⁻ + H₂O**

das störende ClO₃⁻ mit fünfwertigem Chlor (Chlorat) entstünde. Ein Chlorat enthaltendes Produkt sollte jedoch vermieden werden.

Zur Verbesserung der Haltbarkeit des Reaktionsprodukts ist beispielsweise eine Lagerung bei erhöhtem pH-Wert geeignet, beispielsweise bei pH-Wert 10 oder mehr. Die Einstellung dieses pH-Werts kann mit einer geeigneten Base, wie vorstehend zum Herstellungsverfahren beschrieben, vorgenommen werden.

Zur Herstellung von Lösungen, die Dichlorsäuren und/oder die Salze dieser genannten Säuren enthalten ist es überraschenderweise gelungen, die freie peroxochlorige Säure HOOClO, die Dichlorsäuren bzw. die Peroxochlorsäure bei Erniedrigung des pH-Wertes unter 6, z.B. auf pH-Wert 5 oder weniger aus dem erhaltenen Chloritionen enthaltenden Gemisch mit einem Inertgas, wie einem Edelgas, z.B. Argon oder Stickstoff oder auch den Gasen Sauerstoff oder Luft, auszutreiben und aufzufangen. Es hat sich überraschenderweise gezeigt, dass die Ausbeute enorm gesteigert werden kann, wenn man die Gasstrecke sehr kurz hält und flor eine Kühlung des Gasstroms sorgt.

Das bei der Einleitung in Stufe (a) des oben beschriebenen Herstellungsverfahrens entstehende Gemisch enthält zunächst sehr hohe Konzentrationen an Chlorit-Ionen (ClO₂⁻). Der Gehalt an Chlorit kann jedoch durch das "Überleiten" im Gasstrom in eine basische Lösung deutlich reduziert werden. Hierbei werden die Chlorsäuren jeglicher Art als flüchtige Verbindungen in protonierter (neutraler) Form ausgetrieben, die jedoch sehr instabil sind. In der Vorlage befindet sich eine Base, wodurch die Chlorsäuren deprotoniert und die Anionen gebildet werden. Nachdem man die Lösung auf pH 6-8 und nachdem man definierte Mengen an Chlorit beispielsweise in Form von Natriumchlorit zugegeben hat werden dann die Anionen der Dichlorsäuren gebildet.

Es kann beispielsweise in einer Base, wie einer Alkalimetallbase, Erdalkalimetall- oder Zinkbase oder Stickstoffbase, wie Ammoniak oder einem organischen Amin aufgefangen werden. Es ist aber auch möglich die gasförmig anfallenden Säuren in einer Kühlfalle (z.B. bei -100 bis -190 °C) auszufrieren.

Als Gegenionen kommen alle Metallkationen und organische Kationen, wie diejenigen von Stickstoffbasen, insbesondere quaternäre Ammoniumsalze, in Frage. Welche Kationen besonders geeignet sind, ergibt sich aus dem jeweiligen Verwendungszweck. Für pharmazeutische Anwendungen sind insbesondere Erdalkali- oder Alkalimetalle, vorzugsweise Na⁺ oder K⁺, oder Zn²⁺ bevorzugt, bei technischen Anwendungen können auch organische Kationen, wie Kationen von Stickstoffbasen, insbesondere Alkylammoniumkationen, wie Trialkylammoniumkationen, oder vor allem quaternäre Ammoniumkationen eingesetzt werden.

Es ist zweckmäßig und bevorzugt, die erfindungsgemäßen Säuren sowie deren Derivate, Salze und/oder Anionen unter Ausschluss von Licht zu lagern und daraus wässrige Lösungen mit hohen pH-Werten herzustellen, z.B. mit pH-Werten von 10, 11 oder 12 und darüber, insbesondere im Bereich von pH 10 bis pH 13, um lange Lagerfähigkeiten zu erzielen. Aus derartigen Lösungen kann je nach Bedarf die freie Säure, wie vorstehend beschrieben, wieder erhalten und gegebenenfalls in Lösungen mit gewünschtem pH-Wert bzw. in Salze überführt werden.

Die erfindungsgemäßen Dichlorsäuren, bzw. die peroxochlorige Säure, deren jeweiligen Derivate, bzw. Anionen und Salze davon können als solche und insbesondere in wässriger oder wasserhaltiger Lösung als Oxidationsmittel für die verschiedensten medizinischen, kosmetischen, technischen und landwirtschaftlichen Zwecke Verwendung finden.

Beispiele für mögliche Testsysteme finden sich in den eingangs genannten Publikationen und Patentschriften, die hier in dieser Hinsicht durch Bezugnahme aufgenommen werden.

Eine Anwendungsmöglichkeit besteht in der Verwendung als pharmazeutische Präparate (Arzneimittel) bzw. zur Herstellung von Arzneimitteln, die auf jegliche Weise, insbesondere topisch, aber auch parenteral verabreicht werden können. Die Arzneimittel können in üblicher Weise mit üblichen pharmazeutisch verträglichen Trägern und Verdünnungsmitteln formuliert werden.

Die vorliegende Erfindung betrifft ebenfalls pharmazeutische Präparate, die als Wirkstoff die erfindungsgemäßen Dichlorsäuren, bzw. die peroxochlorige Säure, deren Anionen, Derivate oder Salze umfassen und insbesondere zur Behandlung der eingangs genannten Erkrankungen verwendet werden können. Besonders bevorzugt sind Präparate zur enteralen, wie nasalen, bukkalen, rektalen oder insbesondere oralen (vorzugsweise unter Umgehung der Magensäure, z.B. mit magetisaftresistenten Präparaten wie Kapseln oder Lacktabletten), sowie vor allem zur lokalen oder parenteralen, wie intravenösen, intramuskulären oder subkutanen Verabreichung an Wannblüter, insbesondere Menschen. Die Präparate enthalten den Wirkstoff allein oder vorzugsweise zusammen mit einem oder mehreren pharmazeutisch anwendbaren Trägennaterialien. Die Dosierung des Wirkstoffs hängt von der zu behandelnden Krankheit, sowie von Spezies, deren Alter, Gewicht und individuellem Zustand, individuellen pharmakokinetischen Gegebenheiten sowie der Applikationsweise ab. Vorzugsweise liegt die Dosierung für die enteral oder insbesondere parenterale Applikation (beispielsweise durch Infusion oder Injektion) (vorzugsweise am Menschen) im Bereich von 0,01 bis 100 pmol/kg, insbesondere zwischen 0,1 bis 100 pmol, also beispielsweise bei einem Menschen mit einem Körpergewicht von 70 kg bei 1 mg bis 1 g/Tag, insbesondere bei 8,5 mg bis 850 mg/Tag, in einer Dosis oder aufgeteilt auf mehrere Dosen. Für die lokale Applikation liegen bevorzugte Dosisbereiche zwischen 0,1 und 10 insbesondere 0,5 und 5 mL/100 cm² einer 0,1 bis 10 millimolaren Lösung (entsprechend mehr oder weniger für größere oder kleinere Flächen - applizierbar direkt oder unter Verwendung von Verbänden beispielsweise aus getränkter Gaze).

So beschreibt die Anmeldung auch eine Methode zur prophylaktischen und/oder therapeutischen Behandlung von hierin beschriebenen Krankheitszuständen, vorzugsweise zur prophylaklischen und/oder therapeutischen Behandlung von Krankheiten, bei deren Bekämpfung eine Verstärkung der Geweberegeneration, eine Immummodulation, eine Verbesserung der Impfreaktion oder eine Strahlensensibilisierung angezeigt bzw. erfolgreich ist, oder zwei oder mehrere dieser Wirkungen, insbesondere zur Behandlung den Wunderkrankung bei einem Warmblüter, umfassend die Verabreichung von erfindungsgemäßen Dichlorsäuren, bzw. der peroxochlorigen Säure, deren jeweiligen Anionen, Derivate oder Salze in einer gegen die genannten Krankheiten wirksamen Menge an einen Warmblüter, z.B. Menschen, der einer derartigen Behandlung bedarf.

Die Erfindung betrifft auch eine pharmazeutische Zusammensetzung zur prophylaktischen und insbesondere therapeutischen Behandlung von hierin beschriebenen Krankheitszuständen, vorzugsweise zur prophylaktischen oder therapeutischen Behandlung von Krankheiten, bei deren Bekämpfung eine Verstärkung der Geweberegeneration, eine Immunmodulation, eine Verbesserung der Impfreaktion oder eine Strahlensensibilisierung, angezeigt bzw. erfolgreich ist, oder zwei oder mehrere dieser Wirkungen, insbesondere einer Wunderkrankung, vorzugsweise eines Warmblüters, der an einer derartigen Erkrankung leidet, enthaltend erfindungsgemäße Dichlorsäuren oder deren jeweilige Anionen, Derivate oder Salze in einer prophylaktisch oder insbesondere therapeutisch gegen die genannte Erkrankung wirksamen Menge und ein oder mehrere pharmazeutisch verwendbare Trägennaterialien.

Die Anmeldung beschreibt ebenfalls ein Verfahren zur Behandlung von Krankhcitszuständen, vorzugsweise zur prophylaktischen und/oder therapeutischen Behandlung - insbesondere bei einem Warmblüter, insbesondere einem Menschen - von Krankheiten, bei deren Bekämpfung eine Verstärkung der Geweberegeneration, eine Immunmodulation, eine Verbesserung der Impfreaktion und/oder eine Strahlensensibilisierung angezeigt bzw. erfolgreich ist, insbesondere von einer Wunderkrankung, bei einem Warmblüter, umfassend die Verabreichung von erimdungsgemäßen Dichlorsäuren oder deren jeweilige Derivate, Anionen oder Salze in einer gegen die genannten Krankheiten wirksamen Menge an einen Warmblüter, z.B. Menschen, der einer derartigen Behandlung bedarf.

Die Anmeldung beschreibt auch die Verwendung der erfindungsgemäßen Dichlorsäuren und deren Derivate, Anionen oder Salze zur Anwendung in einem Verfahren zur Behandlung des menschlichen oder tierischen Körpers. Die Erfindung betrifft daher auch die Verwendung der erfindungsgemäßen Dichlorsäuren, deren Derivate, Anionen oder Salze zur Herstellung eines Medikaments zur Behandlung des menschlichen oder tierischen Körpers, vorzugsweise zur prophylaktischen und/oder therapeutischen Behandlung - insbesondere bei einem Warmblüter, insbesondere einem Menschen - von Krankheiten, bei deren Bekämpfung eine Verstärkung der Geweberegeneration, eine Immunmodulation, eine Verbesserung der Impfreaktion und/oder eine Strahlensensibilisierung angezeigt bzw. erfolgreich ist, insbesondere zur Wundbehandlung.

Die Erfindung betrifft auch die Verwendung von erfindungsgemäßen Dichlorsäuren deren Anionen, Derivate oder Salze zur Herstellung eines Medikaments zur (kosmenschen) Pflege der Haut, beispielsweise bei Vorliegen einer Neigung zur Entwicklung von Pickeln (z.B. Akne) oder Vorliegen von Pickeln.

Dosiseinbeitsformen sind z.B. Dragées, Tabletten, Ampullen, Vials, Suppositorien oder Kapseln. Weitere Applikationsformen insbesondere für Lösungen von erfindungsgemäßen Dichlorsäuren bzw. peroxochlorige Säure, deren Anionen, Derivate oder Salze, sind z.B. Salben, Crèmes, Pasten, Gele, Schäume, Mundwässer, Tropfen, Spray, und dergleichen. Die Dosiseinheitsformen, z.B. Ampullen, Tabletten oder Kapseln, enthalten vorzugsweise von etwa 0,05 g bis etwa 1,0 g, insbesondere von 8,5 mg bis 850 mg, eines Salzes von erfindungsgemaßen Dichlorsäuren, deren Anionen, Derivate mit üblichen Trägermaterialien.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt.

In einer bevorzugten Ausfübrungsform kann eine 0,05 bis 1 M Lösung eines Dichlorsäuresalzes und/oder eines Salzes von dessen Derivaten in bidestilliertem Wasser bei einem pH gleich oder > 10, vorzugsweise 10 bis 13, insbesondere 12,5, gelöst werden. Diese Lösung wird unmittelbar vor der Verabreichung mit Kochsalz, Natrium- oder Kaliumbicarbonat und bidestilliertem Wasser zur Isotonie auf Konzentrationen von ca. 1 - 5 mM verdünnt und dem physiologischen pH angenähert. Diese Lösung ist für die parenterale, bevorzugt intravenöse Anwendung geeignet.

Für eine bevorzugte Formulierung eines Arzneimittels zur topischen Anwendung werden vorzugsweise die erfindungsgemäßen Dichlorsäuren oder deren Derivate als Salze in bidestilliertem Wasser mit Konzentrationen im unteren millimolaren bzw. oberen mikromolaren Bereich, vorzugsweise im Konzentrationsbercich von 0,5 - 5 mM mit pH gleich oder > 10, insbesondere 10 bis 13, bevorzugt z.B. pH 11,5, gelöst und mit Glycerin, Kochsalz oder einem anderen geeigneten verträglichen, möglichst physiologischen Mittel auf Isotonie eingestellt. Vor der Anwendung wird ein physiologischer pH Wert mit HCl eingestellt. Weitere Zusätze sind möglich. Insbesondere sind bei Abfüllung des Arzneimittels in Plastikbehälter solche Zusatzstoffe geeignet, die Übergangsmetallspuren neutralisieren können, da Übergangsmetalle in den Wänden während Lagerung gelöst werden und eine Zersetzung des Wirkstoffes katalysieren können. Beispiele für solche Zusatzstoffe sind Oligo- oder Polyalkohole, wie Ethylenglykol, Desferrioxamin oder EDTA (z.B. als Dinatrium EDTA). Die so erhaltene Lösung kann direkt auch auf Wunden verabreicht werden.

Die Anionen der erfindungsgemäßen Dichlorsäuren sind stabil, die Säuren selber zerfallen relativ schnell. Eine Arzneiwirkstoffstabifisierung kann deswegen über den pH-Wert erreicht werden. Die Wirkstofflösung kann zur Verbesserung der Verträglichkeit unmittelbar vor Gebrauch durch eine Pufferverdünnung auf einen annähernd physiologischen Wert abgesenkt werden. Dies reicht für die Entfaltung der pharmakologischen Wirkung im ganzen Körper aus, denn diese Wirkung beruht nicht auf der Rezeptor-Liganden-Interaktion eines konventionellen Pharmakons, sondern sie ist, wie bereits dargelegt, mit einer schnell und irreversibel ablaufenden Oxidationsreaktion verknüpft. Deren pharmakologische Wirkung hält an, solange die Zelle und/oder ihre chemisch veränderten Strukturen erhalten bleiben, ist also nicht nach Abdiffundieren einer Wirksubstanz von einem Rezeptor beendet.

Beispiele für Indikationsgebiete, bei denen eine Verstärkung der Geweberegeneration prophylaktisch oder insbesondere therapeutisch zur Behandlung einer Erkrankung erfolgreich ist, sind die Geweberegeneration nach physikalischen Schäden (wie z.B. stumpfe und scharfe Traumen, kurzwelliges Licht, radioaktive Strahlung) und nach chemischer Schädigung (z.B. durch Gewebegifte, wie Lost, Chemotherapeutika). Ein weiteres Anwendungsgebiet in diesem Bereich ist die Verbesserung der Wundheilung insbesondere von hartnäckigen, sogenannten "spontanen" Wunden, die aufgrund eines Grundleidens (beispielsweise Diabetes mellitus, Gefäßleiden, Immunsuppression oder altersbedingt) nicht heilen wollen. Zu derartigen Wunden gehören insbesondere Dekubitus und chronische Beinulcera. Unter Wundbehandlung ist hier insbesondere die Behandlung von Wunden auf der Haut, den Schleimhäuten und in Geweben, wie z.B. Leber, Herzmuskel oder Knochenmark, zu verstehen.

Da es sich bei den erfindungsgemäßen Dichlorsäuren bzw. der peroxochlorigen Säure um definierte Verbindungen handelt, ergeben sich auch keine Schwierigkeiten bei der Arzneimittel-Zulassung.

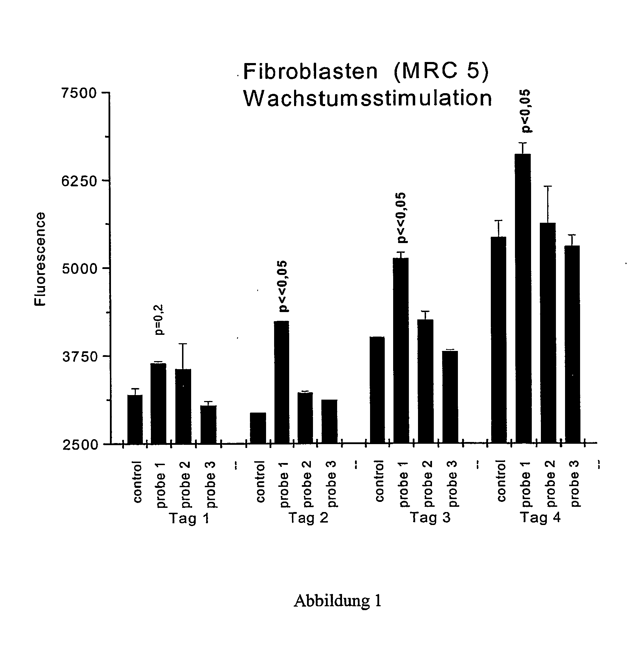

In Abbildung 1 sind die Ergebnisse aus den in Beispiel 3 aufgelisteten Zellkulturversuchen (Wachstumsförderung bei Fibroblasten) dargestellt. Diese wurden mit Wirkstoffkonzentrationen im Kulturmedium von 100 µM Chlorit und 50 µM an reaktivem Chlor (RC = Summe der Anionen aller Dichlorsäuren und der peroxochlorigen Säure) erzielt.

Dabei ist in der Zellkultur eine wachstumsstimulierende Wirkung von 20-25% der Probelösung 1, die sowohl die erfindungsgemäßen Dichlorsäuren als auch Chlorit enthält, deutlich zu erkennen und bezogen auf die Kontrolle auch signifikant höher.

Die Applikation der reinen RC-haltigen oder Chlorit-haltigen Lösungen zeigt genau wie die Kontrolle keinerlei Effekte auf das Wachstumsverhalten der Fibroblasten.

Abbildung 2 zeigt die Titration der in der Lösung vorhandenen Anionen der Peroxo-Säuren (Dichlorsäure, peroxochloriger Säure) zur Ermittlung der Konzentration der Säureanionen. In Abbildung 3 ist die Ableitung der Titrationskurve von Abbildung 2 dargestellt, die zur genauen Konzentrationsbestimmung dient.

In den Abbildungen 4 und 5 sind Beispiele von UV-Spektren dargestellt. Die UV-Absorptionsmessungen erlauben, die Konzentration von vorhandenem Chlorit zu bestimmen und zeigen eventuell vorhandenes gelöstes freies Chlordioxid an.

Abbildung 6 zeigt ein Massenspektrum der Produktlösung, wobei das Peroxochlorit (Masse 83,2) und das Anion der Dichlorsäure (Masse 189) nachgewiesen wurde.

In Abbildung 7 ist das Ergebnis einer Ionenchromatografie dargestellt. Die Retentionszeiten von Vergleichssubstanzen sind in Beispiel 4 -Teil 5 angegeben. Die Dichlorsäure wird bei 19,77 min detektiert, dagegen ist kein Chlorat (ClO₃⁻) nachweisbar, was Chlorat als Verursacher des Peaks im Massenspektrum bei 82,3 in Abbildung 6 ausschließt.

Abbildung 8 zeigt eine Absterbekinetik mit drei Bakterienstämmen, *E*. *coli, S. aureus* und *P*. *aeruginosa.* Bei allen drei Stämmen wurde mit der Produktlösung eine bakterizide Wirkung nach DIN 58940 erzielt.

Die nachfolgenden Beispiele erläutern die Erfindung näher, sollen jedoch keinesfalls einschränkend verstanden werden.

### Beispiel 1: Herstellung der Dichlorsäuren

Zu einer Lösung von 100 g wasserfreiem Natriumchlorit in 200 mL Wasser gibt man unter Rühren vorsichtig tropfenweise Schwefelsäure (96%-ig) zu. Mit einem kräftigen Gasstrom (Ar, N₂ oder O₂ bzw. CO₂-freie Luft) wird das entstehende Chlordioxid ausgetrieben. Der Gasstrom muss so stark sein, dass der Gehalt an ClO₂ nicht über 5 Prozent steigt (Explosionsgefahr). Der ClO₂-haltige Gasstrom wird, um elementares Chlor abzufangen, über drei hintereinander geschaltete Waschflaschen, die mit je 30 mL einer 2 M NaClO₂-Lösung vom pH 11 beschickt sind, in eine Lösung von 15 mL 30%-igem Wasserstoffperoxid in 35 mL Wasser, die zuvor durch Zugabe von 4M Natronlauge auf pH 12 gebracht ist, eingeleitet. An Stelle von Wasserstoffperoxid kann auch eine Lösung von Natriumperborat oder Natriumpercarbonat oder einer anderen Peroxoverbindung wie z.B. das H₂O₂-Addukt des Harnstoffs verwendet werden. Während der Gaseinleitung wird der pH-Wert mit einer Glaselektrode kontrolliert. Durch Zugabe von 4M NaOH wird der pH-Wert im Verlauf der Reaktion bei 12 gehalten. Das vorgelegte Hydroperoxid bzw. die vorgelegte Peroxoverbindung ist verbraucht, wenn die Gaseinleitung zu einer bleibenden Gelbfärbung führt. Mit einem Tropfen der Lösung des Oxidationsmittels (z.B. H₂O₂) wird die gelbe Lösung anschließend wieder entfärbt.

Die reaktives Chlor enthaltende Lösung wird unter Rühren zu einer Lösung von 500 g Zitronensäure in 3 Liter Wasser getropft, die zuvor mit 2 M Natronlauge auf pH 4,5 eingestellt ist. Während der Zugabe wird die gebildete reaktive Chlorverbindung mit einem kräftigen Gasstrom (N₂ oder O₂) ausgetrieben. Der Gasstrom ist vorzugsweise zu kühlen. Die Schlauchverbindungen sollten möglichst kurz sein. Das Gas wird beispielsweise in drei hintereinandergeschalteten Waschflaschen, die mit je 50 mL 0,1 M NaOH beschickt sind, aufgefangen.

Die Inhalte der Waschflaschen werden vereinigt und bei pH > 10 gehalten.

Zur Bildung der erfindungsgemäßen Dichlorsäuren wird der pH auf 7 mit beispielsweise Salzsäure eingestellt, und es wird ein 10facher molarer Überschuß an Natriumchlorit zugegeben. Zur Lagerung ist es dann bevorzugt, wenn ein pH-Wert von etwa größer gleich 10 bis etwa 13 eingestellt wird.

Der Gesamtgehalt an reaktiven Chlor Anionen wird durch potentiometrische Titration mit 0,1 M HCl in einer dem Fachmann wohl bekannten Weise bestimmt.

Die gebildeten Dichlorsäuren liegen in Lösung im Gemisch mit einer definierten Menge an Chlorit sowie weiteren reaktiven Chlorverbindungen vor.

Das Vorhandensein der Dichlorsäuren wird mit Ramanspektroskopie nachgewiesen.

### Beispiel 2: Kultivierung von MRC-5-Fibroblasten

### Lösungen:

| | |
|---|---|
| Kulturmedium für MRC-5: | |
| | 89 mL IF-Basalmedium |
| | 10 mL FCS (Fötales Kälber Serum) |
| | 1 mL L-Glutaminstammlösung |
| IF-Basalmedium | |
| | Das IF-Basalmedium ist eine 1:1 1 Mischung von IMDM (Iscove's Modifiziertes Dulbecco's Medium) und Ham's F12-Medium. |
| L-Glutamin-Stammlösung | |
| | 200 mM L-Glutamin werden in IF-Basalmedium gelöst und sterilfiltriert. |

### Kultivierung:

Die verwendete Zelllinie MRC-5 wird in ungelatinierten Zellkulturgefäßen ausgesät. Die anschließende Kultivierung erfolgt im Brutschrank bei 37°C und 5 Vol.-% CO₂ in wasserdampfgesättigter Atmosphäre. Ein Wechseln des Kulturmediums erfolgt jeden zweiten bis dritten Tag, und nach Erreichen der Konfluenz werden die Zellen mit einer Teilungsrate von 1:5 bis 1:10 passagiert.

### Beispiel 3: Zellbiologischer Test zur Wirkstoffprüfung

### Lösungen:

| | |
|---|---|
| Kulturmedium für MRC-5: | |
| | 89 mL IF-Basalmedium |
| | 10 mL FCS (Fötales Kälber Serum) |
| | 1 mL L-Glutaminstammlösung |
| serumreduziertes Kulturmedium für MRC-5: | |
| | 98 mL IF-Basalmedium |
| | 1 mL FCS (Fötales Kälber Serum) |
| | 1 mL L-Glutaminstammlösung |
| PBS | (Phosphate Buffered Saline): |
| | 140 mM NaCl, 3 mM KCl, 8 mM Na₂HPO₄ und 1,5 mM KH₂PO₄ werden in Wasser gelöst, wobei sich ein pH-Wert von 7,2 - 7,4 einstellt. Die erhaltene Lösung wird durch Autoklavieren sterilisiert. |
| Zelllysepuffer | |
| | 0,04% SDS (Stammlösung 10% SDS) |
| | 2x SSC (Stammlösung 20x SSC) |
| | für 25 mL fertigen Zelllysepuffer werden 5,0 mL 20x SSC und 100 µL 10% |
| | SDS auf 25 mL mit PBS aufgefüllt. |
| DAPI-Lösung | |
| | 2 µM DAPI in PBS |

### Kultivierung:

Die verwendeten Zellen MRC-5 werden mit 400 Zellen/cm² in einer 24-Loch Zellkulturplatte ausgesät. Die anschließende Kultivierung erfolgt im Brutschrank bei 37°C und 5 Vol.-% CO₂ in wasserdampfgesättigter Atmosphäre. Nach 24 Stunden Vorkultivierung wir das Kulturmedium abgesaugt und die Zellen werden mit PBS gewaschen. Es erfolgt ein Wechseln des Kulturmediums auf serumreduziertes Kulturmedium und die zu testenden Wirkstoffe (eine Übersicht zeigt nachfolgende Tabelle) werden zugegeben.

Nach 24, 48 und 72 Stunden wird die Proliferation der Zellen durch Quantifizierung der zellulären DNS nach DAPI-Färbung im Fluorometer (Novostar -Firma BMG Labtechnologies) bestimmt. Dabei ist eine gesteigerte Fluoreszenz in den Proben mit einer Proliferation der Zellen gleichzusetzen.

Die zu messende Platte wird 1 mal mit 500 µl PBS pro Loch gewaschen und danach werden in jedes Loch 250 µl PBS vorgelegt. Es werden 250 µl Lysispuffer zugefügt und die Zellen werden für 30 min bei RT unter leichtem Schütteln auf dem Schüttler lysiert. Danach werden 500 µl DAPI-Lösung zugefügt und das ganze weitere 10 min bei Raumtemperatur stehen gelassen.

Die Platte wird bei 355 nm ex. und 460 nm em. im Novostar gemessen. Üblicherweise wird bei einer Gain-Einstellung von 1400-1600 gearbeitet.

Die Mehrfachbestimmungen werden gemittelt und die Fehlerwerte berechnet. Die ermittelten Daten werden graphisch ausgewertet.

Es werden die folgenden Stammlösungen eingesetzt:

**Tabelle 2 Kombination der verwendeten Wirkstoffe**

| **[Konzentration der Stammlösungen]** | | | |
|---|---|---|---|
| Bezeichnung | Gehalt an RC [mM] | Gehalt an Chlorit [mM] | Bemerkung |
| Kontrolle | -- | -- | serumreduziertes Kulturmedium |
| Probe 1 | 40 | 6 | in serumreduziertem Kulturmedium |
| Probe 2 | 40 | -- | in serumreduziertem Kulturmedium |
| Probe 3 | -- | 6 | in serumreduziertem Kulturmedium |

### Beobachtete Wachstumsstimulation bei Fibroblasten:

Zur Verwendung in der Zellkultur werden die Lösungen vor dem Einsatz in den angegebenen Kulturmedien verdünnt. Die in Abbildung 1 dargestellten Ergebnisse wurden mit

Wirkstoffkonzentrationen von 100 µM Chlorit und 50 µM des RC (= Gemisch der erfindungsgemäßen Dichlorsäuren und der peroxochlorigen Säure, bzw. deren Anionen) erzielt.

Dabei ist eine wachstumsstimulierende Wirkung von 20 -25% der Probelösung 1, die sowohl RC als auch Chlorit enthält, deutlich zu erkennen und bezogen auf die Kontrolle auch signifikant höher.

### Beispiel 4: Analytische Bestimmungen der aus Beispiel 1 erhaltenen Lösung:

### 1) pH-Messung:

Der pH-Wert wird mit der Einstab-Glaselektrode bestimmt. Abhängig vom pH-Wert ist der Produktgehalt und die Lage des Gleichgewichtes.

### 2) Titration mit 0,1 M HCl:

Die Titration dient beispielsweise der quantitativen Bestimmung des Dichlorsäure-Gehaltes oder auch des Gehalts an peroxochloriger Säure oder des Peroxochlorats.

Je 1 mL der Produktlösung werden mit 0,1 M Salzsäure potentiometrisch titriert. Es werden Titrationskurven (pH vs. mL 0,1 M HCl) aufgenommen. Aus dem in der Ableitung der Titrationskurve ermittelten Säureverbrauch zwischen pH 8,5 und 4,5 wird der Gehalt an Anionen der entsprechenden Säuren in Summe bestimmt.

In einem typischen Ergebnis ergibt 1 mL Produktlösung einen Verbrauch von 0,72 mL 0,1 M HCl und damit eine Konzentration von 0,072 M.

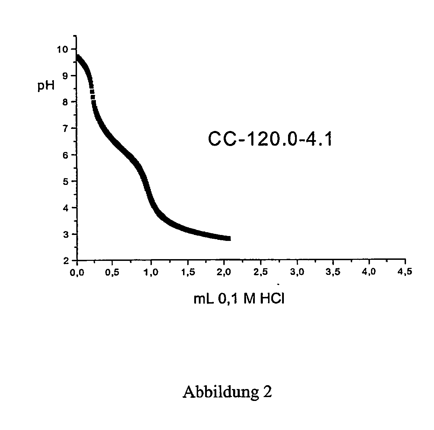

Eine aufgenommene Titrationskurve zeigt Abbildung 2:

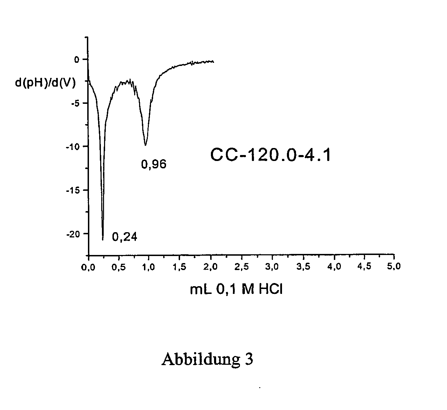

Die Ableitung der Titrationskurve und Ermittlung der Konzentration zeigt Abbildung 3.

### 3) UV-vis-Absorptionsspektrum:

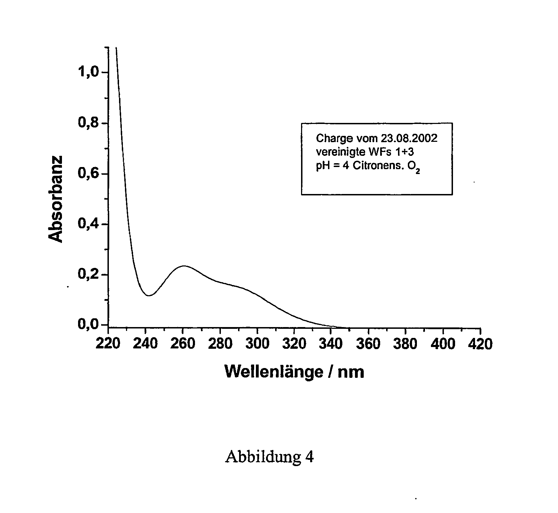

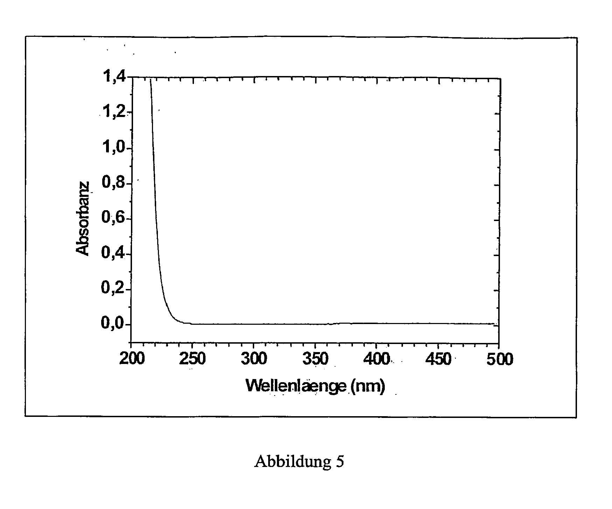

Die Messung des UV-Spektrums dient der Quantifizierung des Gehaltes an Chlorit in der Produktlösung. Zum Vergleich sind in Abbildung 4 bzw. 5 Spektren einer Chlorit-haltigen und einer Chlorit-freien Produktösung gezeigt. Bei 260 nm liegt das Chlorit Signal; Chlordioxid, das aus dem Prozeß stammt, zeigt ein Signal bei 360 nm.

In 1 cm-Quarz-Küvetten werden die Absorbanz-Werte bei 260 nm und 500 nm bestimmt. Aus der Differenz A260 - A500 und mit Hilfe des Extinktionskoeffizienten für Chlorit von ∈260 nm =140 M⁻¹ cm⁻¹ bei 260 nm lässt sich der Gehalt an ClO₂⁻-Ionen bestimmen.

Eine Absorption bei 360 nm deutet auf freies Chlordioxid hin (∈360 nm =1260 M⁻¹cm⁻¹).

### 4) Massenspektroskopie

Die ESI Massenspektrometrie wurde mit einem Bruker Esquire-LC Spektrometer im Standard MS-Modus durchgeführt. Die Probe war eine wässrige Produktösung, die vor der Messung mit Methanol verdünnt wurde. Der verwendete Scan-Bereich lag zwischen 30 m/z und 400 m/z, mit Capillary Exit -65 Volt und Skim -15 Volt; das Spektrum repräsentiert einen Durchschnittswert von 50 Messungen.

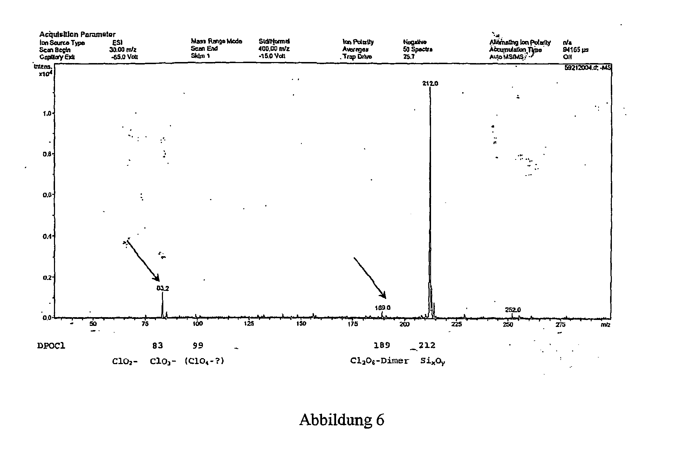

Der rechte Pfeil in Abbildung 6 deutet auf das Signal der Dichlorsäure (Summenformel: Cl₂O₆²⁻), der linke zeigt das Signal der bislang unbekannten Peroxochlorit-Spezies (Summenformel: ClO₃⁻).

### 5) Ionenchromatografie

Alle Analysen wurden mit einem modularen Ionenchromatographie-System der Fa. Metrohm durchgeführt.

| | |
|---|---|
| Pumpe: | Metrohm IC 709 Pump |
| Detektor: | Metrohm 732 IC Detektor |
| Suppressor: | Metrohm 753 Suppressor Module |
| Säule: | Metrosep A 250 |
| Flussrate: | 1 ml/min |
| Injektionsvolumen: | 20 µL |
| Eluent: | 1mM NaOH |

Von Referenzsubstanzen wurden jeweils unmittelbar vor der Messung frische Lösungen bekannter Konzentration erstellt, die dann mit der oben beschriebenen Methode mit dem angegebenen Eluenten gemessen wurden.

Retentionszeiten der Referenzsubstanzen:

| Substanz | Retentionszeit [min] |
|---|---|
| NaCl | 13,21 |
| NaClO₂ | 12,30 |
| NaClO₃ | 16,26 |
| NaClO₄ | 4,36 |
| NaOH | 17,32 |
| Na₂CO₃ | 21,98 |
| Na₂Cl₂O₆ | 19,77 |

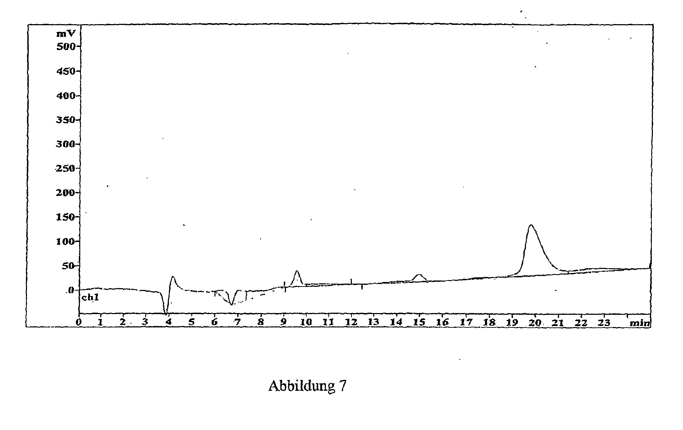

Abbildung 7: In der Ionenchromatographie liefert die Dichlorsäure einen typischen Peak bei einer Retentionszeit von 19,77 min. Keine der bekannten Referenzsubstanzen konnte nachgewiesen werden. Die Ionenchromatografie bestätigt die Beobachtung aus der Massenspektroskopie. Ein Chlorat-typischer Peak (NaClO₃, Retentionszeit 16,26 min) ist in der nach Beispiel 1 hergestellten Lösung nicht nachweisbar. Daher kann es sich bei dem Peak der Summenformel ClO₃⁻ in der Massenspektroskopie (Abb. 6, Masse 83,2) nur um die neue peroxochlorige Säure bzw. deren Anion handeln.

### Beispiel 5: Bakterizide Wirkung der aus Beispiel 1 erhaltenen Lösung:

Absterbekinetik nach DIN 58940
Lösung nach Beispiel 1 wurde als 1:10-Verdünnung eingesetzt.
Test-Organismen: *Escherichia coli* ATCC 25922, *Pseudomonas aeruginosa* ATCC 27853, *Staphylococcus aureus* ATCC 29213
Nährmedium: Caseinpepton- Sojapepton (Ph.Eur.2.6.12)
Dauer Inkubation der Bakterien: 18 h +/- 1 h

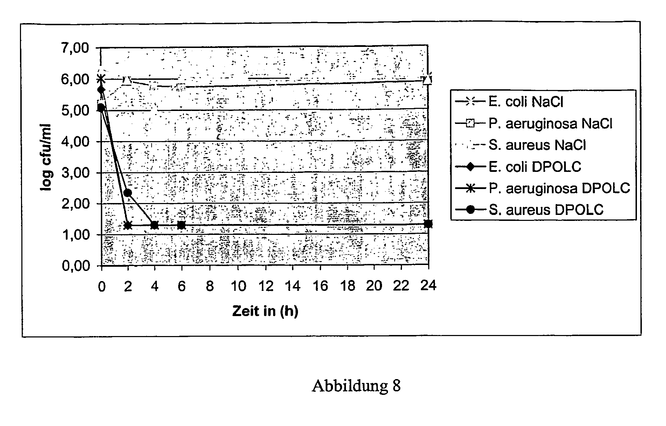

Das Ergebnis ist in der folgenden Tabelle 3 sowie in Abbildung 8 gezeigt. Damit ist die bakterizide Wirkung der verwendeten Lösung nach DIN 58940 nachgewiesen.

**Tabelle 3:**

| | E. coli NaCl | | | P. aeruginosa NaCl | | | S. aureus NaCl | | |
|---|---|---|---|---|---|---|---|---|---|
| Zeit (h) | cfu/ml | log cfu/ml | | cfu/ml | log cfu/ml | | cfu/ml | log cfu/ml | |
| 0 | 1,04E+06 | 6,02 | 6,02 | 1,58E+05 | 5,20 | 5,20 | 1,98E+06 | 6,30 | 6,30 |
| 2 | 5,60E+05 | 5,75 | 5,75 | 8,60E+05 | 5,93 | 5,93 | 9,00E+05 | 5,95 | 5,95 |
| 4 | 5,00E+05 | 5,70 | 5,70 | 6,00E+05 | 5,78 | 5,78 | 1,12E+05 | 5,05 | 5,05 |
| 6 | 8,60E+05 | 5,93 | 5,93 | 5,40E+05 | 5,73 | 5,73 | 9,80E+05 | 5,99 | 5,99 |
| 24 | 1,04E+06 | 6,02 | 6,02 | 7,60E+05 | 5,88 | 5,88 | 8,00E+05 | 5,90 | 5,90 |
| | | | | | | | | | |
| | | | | | | | | | |

| Ansätze mit 300µg/ml DPOLC | | | | | | | | | |
|---|---|---|---|---|---|---|---|---|---|
| | E. coli DPOLC | | | P. aeruginosa DPOLC | | | S. aureus DPOLC | | |
| Zeit (h) | cfu/ml | log cfu/ml | | cfu/ml | log cfu/ml | | cfu/ml | log cfu/ml | |
| 0 | 4,60E+05 | 5,66 | 5,66 | 1,04E+06 | 6,02 | 6,02 | 1,22E+05 | 5,09 | 5,09 |
| 2 | <20 | <1,30 | 1,30 | <20 | <1,30 | 1,30 | 2,20E+02 | 2,34 | 2,34 |
| 4 | <20 | <1,30 | 1,30 | <20 | <1,30 | 1,30 | <20 | <1,30 | 1,30 |
| 6 | <20 | <1,30 | 1,30 | <20 | <1,30 | 1,30 | <20 | <1,30 | 1,30 |
| 24 | <20 | <1,30 | 1,30 | <20 | <1,30 | 1,30 | <20 | <1,30 | 1,30 |

## Patentansprüche

1. Verfahren zur Herstellung von wässrigen Lösungen von reaktiven Chlorverbindungen, bevorzugt von Dichlorsäuren und peroxochloriger Säure, **dadurch gekennzeichnet, dass** man
(a) Chlordioxid mit einer wässrigen oder wasserhaltigen Losung von Wasserstoffperoxid oder einem anderen Hydroperoxid oder Peroxid bei einem pH-Wert von >= 6,5 umsetzt,
(b) den pH-Wert durch Zusatz einer Säure auf 3 bis 6 erniedrigt,
(c) die gasförmige freie reaktive Chlorverbindung, bevorzugt die Dichlorsäure bzw. die peroxochlorige Säure mit einem gekühlten Gas austreibt und in einer basischen Lösung mit einem pH-Wert von >10 auffängt, und
(d) die aufgefangene reaktive Chlorvorbindung, bevorzugt die Dichlorsäure bzw. die peroxochlorige Säure mit einen, bis zu 100fachen Überschuss, bevorzugt bis zu 10fachen Überschuss Chlorit bei einem pH-Wert von 6 bis 8, bevorzugt etwa 7 inkubiert.

2. Wässrige Lösungen von reaktiven Chlorverbindungen erhältlich nach Anspruch 1, umfassend Dichlorsäuren mit der Bruttoformel H₂Cl₂O₆ und deren Derivate, Anionen, oder Salze, mit den Strukturformeln der Anionen 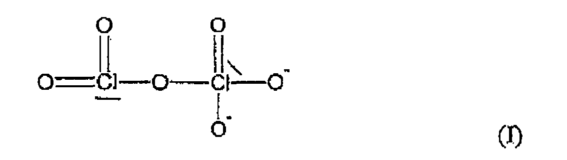 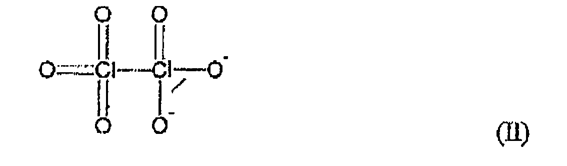 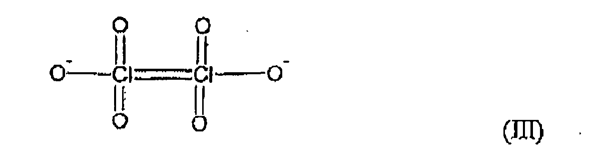 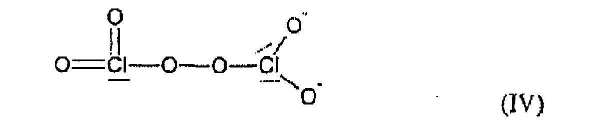 wobei die Dichlorsäuren der Anionen der Strukturfonneln I- III besonders bevorzugt sind.

3. Wässrige Lösung nach Anspruch 2, umfassend peroxochlorige Säure oder deren Anionen, Derivate, oder Salze mit der Strukturformel O=ClOOH bzw. O=ClOO⁻.

4. Wässrige Lösung nach Anspruch 2 oder 3 mit einer Konzentration an Dichlorsäuren und deren Derivate, Anionen, oder Salze nach Anspruch 2 bzw, an peroxochloriger Säure bzw. deren Anionen, Derivate, oder Salze nach Anspruch 3 von mindestens 0,01 M, bevorzugt mindestens 0,025 M, besondere bevorzugt mindestens 0,05 M, ganz besonders bevorzugt mindestens 0,075 M, noch mehr bevorzugte mindestens 0,1 M und am meisten, bevorzugt mindestens 0,5 M.

5. Verfahren nach Anspruch 1, **dadurch gekennzeichnet, dass** man die freie reaktive Chlorverbindung, bevorzugt die Dichlorsäure nach Anspruch 2 oder die peroxochlorige Säure nach Anspruch 3, oder deren Derivate in einer Kühlfalle auftängt.

6. Verfahren nach Anspruch 1, **dadurch gekennzeichnet, dass** man die freie reaktive Chlorverbindung, bevorzugt die Dichlorsäure, peroxochlorige Säure oder deren Derivate aus Stufe (d) in eine wässrige alkalische Lösung einleitet, bevorzugte mit einem pH-Wert von größer gleich etwa 10 bis etwa 13.

7. Verfahren nach Anspruch 6, **dadurch gekennzeichnet, dass** man als Base eine Alkalimetalle, Erdalkalimetall-, Zink- oder Stickstoffbase oder ein Hydroxid eines quaternären Ammoniumsalzes verwendet.

8. Verfahren nach Anspruch 1, **dadurch gekennzeichnet, dass** man die aus Stufe (d) erhaltenen Lösungen durch Erhöhen des pH-Wertes stabilisiert.

9. Pharmazeutisches Präparat, umfassend mindestens eine wässrige Lösung nach einem der Ansprüche 2 bis 4.

10. Pharmazeutisches Präparat nach Anspruch 9, **dadurch gekennzeichnet, dass** es zur parenteralen oder topischen Verabreichung formuliert ist.

11. Verwendung der wässrigen Lösung nach einem, der Ansprüche 2 bis 4 als Oxidationsmittel, Konservierungsmittel und/oder Bleichmittel.

12. Verwendung der wässrigen Lösung nach einem der Ansprüche 2 bis 4 zur Herstellung eines Medikamentes zur prophylaktischen und/oder therapeutischen Behandlung - insbesondere bei einem Warmblüter, insbesondere einem Menschen - von Krankheiten, bei deren Bekämpfung eine Verstärkung der Geweberegeneration, eine Immunmodulation, eine Verbesserung der Impfreaktion und/oder eine Strahlensensibilisierung angezeigt bzw. erfolgreich ist, insbesondere zur Wundbehandlung.

13. Pharmazeutische Zusammensetzung zur prophylaktischen und insbesondere therapeutischen Behandlung von Erkrankungen, vorzugsweise zur prophylaktischen und/oder therapeutischen Behandlung - insbesondere bei einem Warmblüter, insbesondere einem Menschen - von Krankheiten, bei deren Bekämpfung eine Verstärkung der Geweberegeneration, eine Immunmodulation, eine Verbesserung der Impfreaktion oder eine Strahlensensibilisierung angezeigt bzw, erfolgreich ist, insbesondere einer Wunderkrankung, eines Warmblüters, der an einer derartigen Erkrankung leidet, enthaltend die wässrige Lösung nach einem der Ansprüche 2 bis 4 in einer prophylaktisch oder insbesondere therapeutisch gegen die genannte Erkrankung wirksamen Menge und ein oder mehrere pharmazeutisch verwendbare Trägermaterialien.

14. Verwendung der wässrigen Lösung nach einem der Ansprüche 2 bis 4 zur Herstellung eines Medikamentes zur Desinfektion, bevorzugt unter Zumischung von Chlorit.

## Claims

1. Process for the preparation of aqueous solutions of reactive chlorine compounds, preferably of dichloric acids and peroxochlorous acid, **characterized in that**
(a) chlorine dioxide is reacted with an aqueous or water-containing solution of hydrogen peroxide or another hydroperoxide or peroxide at a pH of >= 6.5,
(b) the pH is lowered to 3 to 6 by addition of an acid,
(c) the gaseous free reactive chlorine compound, preferably the dichloric acid or the peroxochlorous acid, is expelled using a cooled gas and collected in a basic solution having a pH of > 10, and
(d) the collected reactive chlorine compound, preferably the dichloric acid or the peroxochlorous acid, is incubated with an up to 100-fold excess, preferably up to 10-fold excess, of chlorite at a pH of 6 to 8, preferably about 7.

2. Aqueous solutions of reactive chlorine compounds obtainable according to Claim 1, comprising dichloric acids having the empirical formula H₂Cl₂O₆ and derivatives, anions or salts thereof, having the structural formulae of the anions 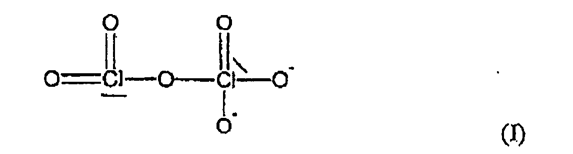 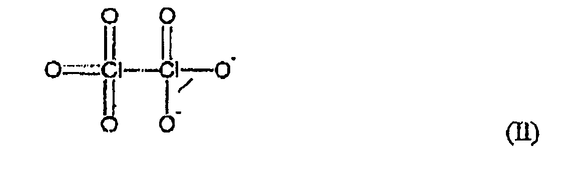 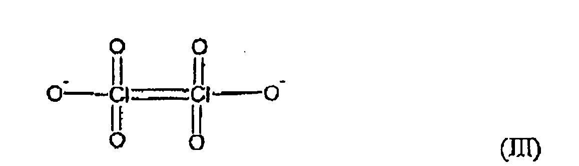 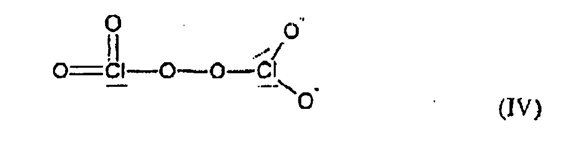 where the dichloric acids of the anions of the structural formulae I-III are particularly preferred.

3. Aqueous solution according to Claim 2, comprising peroxochlorous acid or anions, derivatives or salts thereof having the structural formula O=ClOOH or O=ClOO⁻.

4. Aqueous solution according to Claim 2 or 3 having a concentration of dichloric acids and derivatives, anions or salts thereof according to Claim 2 or of peroxochlorous acid and anions, derivatives or salts thereof according to Claim 3 of at least 0.01 M, preferably at least 0.025 M, particularly preferably at least 0.05 M, very particularly preferably at least 0.075 M, still more preferably at least 0.1 M and most preferably at least 0.5 M.

5. Process according to Claim 1, **characterized in that** the free reactive chlorine compound, preferably the dichloric acid according to Claim 2 or the peroxochlorous acid according to Claim 3, or derivatives thereof is collected in a cold trap.

6. Process according to Claim 1, **characterized in that** the free reactive chlorine compound, preferably the dichloric acid, peroxochlorous acid or derivatives thereof from step (d), is passed into an aqueous alkaline solution, preferably having a pH of greater than or equal to about 10 to about 13.

7. Process according to Claim 6, **characterized in that** the base used is an alkali metal, alkaline-earth metal, zinc or nitrogen base or a hydroxide of a quaternary ammonium salt.

8. Process according to Claim 1, **characterized in that** the solutions obtained from step (d) are stabilized by increasing the pH.

9. Pharmaceutical preparation, comprising at least one aqueous solution according to one of Claims 2 to 4.

10. Pharmaceutical preparation according to Claim 9, **characterized in that** it is formulated for parenteral or topical administration.

11. Use of the aqueous solution according to one of Claims 2 to 4 as oxidant, preservative and/or bleaching agent.

12. Use of the aqueous solution according to one of Claims 2 to 4 for the preparation of a medicament for the prophylactic and/or therapeutic treatment - in particular in a warm-blooded animal, in particular a human - of diseases in the combating of which an increase in tissue regeneration, an immunomodulation, an improvement in vaccine reaction and/or a radiation sensitization is indicated or successful, in particular for wound treatment.

13. Pharmaceutical composition for the prophylactic and in particular therapeutic treatment of diseases, preferably for the prophylactic and/or therapeutic treatment - in particular in a warm-blooded animal, in particular a human - of diseases in the combating of which an increase in tissue regeneration, an immunomodulation, an improvement in vaccine reaction or a radiation sensitization is indicated or successful, in particular a wound disease, of a warm-blooded animal which is suffering from a disease of this type, comprising the aqueous solution according to one of Claims 2 to 4 in an amount which is prophylactically or in particular therapeutically effective against the said disease, and one or more pharmaceutically usable excipient materials.

14. Use of the aqueous solution according to one of Claims 2 to 4 for the preparation of a medicament for disinfection, preferably with admixture of chlorite.

## Revendications

1. Procédé de préparation de solutions aqueuses de composés de chlore réactifs, préférablement d'acides dichloriques et d'acide peroxochloreux, **caractérisé en ce que**
(a) du dioxyde de chlore est réagi avec une solution, aqueuse ou contenant de l'eau, de peroxyde d'hydrogène ou d'un autre hydroperoxyde ou peroxyde à un pH >= 6,5,
(b) le pH est réduit jusqu'à une valeur de 3 à 6 par addition d'un acide,
(c) le composé de chlore réactif libre gazeux, préférablement l'acide dichlorique ou l'acide peroxochloreux, est expulsé à l'aide d'un gaz refroidi et récupéré dans une solution basique ayant un pH > 10, et
(d) le composé de chlore réactif récupéré, préférablement l'acide dichlorique ou l'acide peroxochloreux, est incubé avec un excès jusqu'à 100 fois, préférablement un excès jusqu'à 10 fois, de chlorite à un pH allant de 6 à 8, préférablement d'environ 7.

2. Solutions aqueuses de composés de chlore réactifs obtenables selon la revendication 1, comprenant des acides dichloriques répondant à la formule empirique H₂Cl₂O₆, et des dérivés, anions ou sels de ceux-ci, répondant aux formules structurelles des anions 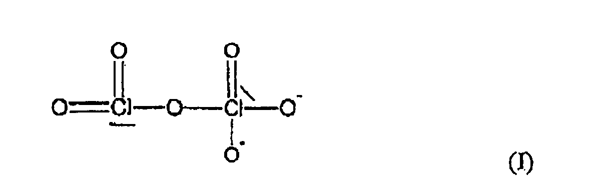 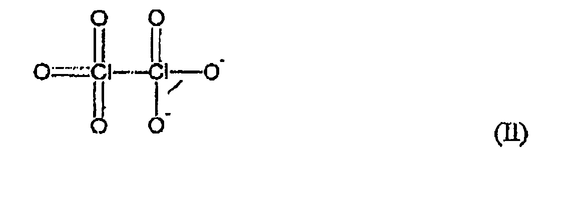 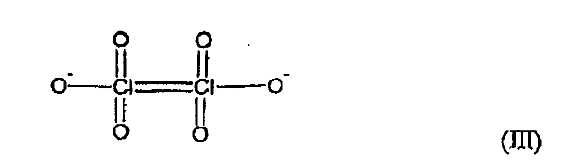 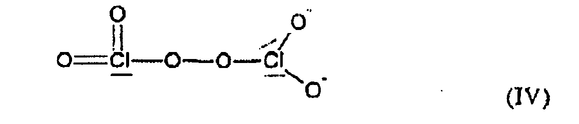 où les acides dichloriques des anions de formules structurelles I-III sont particulièrement préférés.

3. Solution aqueuse selon la revendication 2, comprenant de l'acide peroxochloreux ou des anions, dérivés ou sels de celui-ci répondant à la formule structurelle O=CIOOH ou O=ClOO⁻.

4. Solution aqueuse selon la revendication 2 ou 3, ayant une concentration en acides dichloriques et en dérivés, anions ou sels de ceux-ci selon la revendication 2 ou en acide peroxochloreux et en anions, dérivés ou sels de celui-ci selon la revendication 3 d'au moins 0,01 M, préférablement d'au moins 0,025 M, particulièrement préférablement d'au moins 0,05 M, très particulièrement préférablement d'au moins 0,075 M, encore plus préférablement d'au moins 0,1 M et tout préférablement d'au moins 0,5 M.

5. Procédé selon la revendication 1, **caractérisé en ce que** le composé de chlore réactif libre, préférablement l'acide dichlorique selon la revendication 2 ou l'acide peroxochloreux selon la revendication 3, ou des dérivés de ceux-ci est récupéré dans un piège froid.

6. Procédé selon la revendication 1, **caractérisé en ce que** le composé de chlore réactif libre, préférablement l'acide dichlorique, l'acide peroxochloreux ou des dérivés de ceux-ci issu de l'étape (d), est passé dans une solution alcaline aqueuse, préférablement ayant un pH d'une valeur supérieure ou égale à d'environ 10 à environ 13.

7. Procédé selon la revendication 6, **caractérisé en ce que** la base utilisée est une base de métal alcalin, de métal alcalino-terreux, de zinc ou d'azote ou un hydroxyde d'un sel d'ammonium quaternaire.

8. Procédé selon la revendication 1, **caractérisé en ce que** les solutions obtenues dans l'étape (d) sont stabilisées par une augmentation de pH.

9. Préparation pharmaceutique, comprenant au moins une solution aqueuse selon l'une des revendications 2 à 4.

10. Préparation pharmaceutique selon la revendication 9, **caractérisée en ce qu'**elle est formulée pour une administration parentérale ou topique.

11. Utilisation de la solution aqueuse selon l'une des revendications 2 à 4 comme agent oxydant, conservateur et/ou de blanchiment.

12. Utilisation de la solution aqueuse selon l'une des revendications 2 à 4 pour la préparation d'un médicament destiné au traitement prophylactique et/ou thérapeutique - en particulier chez un animal à sang chaud, en particulier un être humain - de maladies pour la lutte contre lesquelles une augmentation de régénération tissulaire, une immunomodulation, une amélioration de la réaction à un vaccin et/ou une sensibilisation aux rayonnements est indiquée ou fructueuse, en particulier dans le cas de traitement de plaies.

13. Composition pharmaceutique destinée au traitement prophylactique et en particulier thérapeutique de maladies, préférablement destinée au traitement prophylactique et/ou thérapeutique - en particulier chez un animal à sang chaud, en particulier un être humain - de maladies pour la lutte contre lesquelles une augmentation de régénération tissulaire, une immunomodulation, une amélioration de la réaction à un vaccin ou une sensibilisation aux rayonnements est indiquée ou fructueuse, en particulier une maladie de type plaie, d'un animal à sang chaud souffrant d'une maladie de ce type, comprenant la solution aqueuse selon l'une des revendications 2 à 4 en une quantité qui est efficace sur le plan prophylactique ou en particulier sur le plan thérapeutique, contre ladite maladie, et une ou plusieurs substances excipients pharmaceutiquement utilisables.

14. Utilisation de la solution aqueuse selon l'une des revendications 2 à 4, pour la préparation d'un médicament destiné à la désinfection, préférablement en mélange avec du chlorite.
